# EUROPEAN PATENT APPLICATION

(11) **EP 1 547 556 A1**
(43) Date of publication of application: **29.06.2005**
(21) Application number: 04380251.1
(22) Date of filing: 03.12.2004
(51) Int. Cl.: A61F 13/494

(54) **Disposable absorbent article with outwardly disposed outer cuffs**

(30) Priority: 19.12.2003 MX PA04000090; 28.06.2004 MX PA04006369
(71) Applicant: Grupo P.I. Mabe, S.A. de C.V., 72230 Puebla, Estado de Puebla (MX)
(72) Inventor: Corona Carlos, Alberto, Col. La Loma 72230 Puebla (MX); Canales Espinosa de Los Monteros, Carlos, Col. La Loma 72230 Puebla (MX); Sanchez Fernandez, Lucia, Col. La Loma 72230 Puebla (MX); Gonzalez Martinez, Raul, Col. La Loma 72230 Puebla (MX)
(74) Representative: Gil Vega, Victor

(57) **Abstract**

A disposable absorbent article, comprising a pervious topsheet, an impervious backsheet, an absorbent core disposed between the topsheet and the backsheet, the absorbent core having two longitudinal edges and two transverse edges, elastic waist bands and a fastening system; the article has a front zone, a back zone and a crotch zone, as well as two longitudinal edges and two transverse edges. The article also has two anti-leakage cuffs disposed over the topsheet, the cuffs having a fixed edge attached, by means of a first joint, along the entire length of the diaper over the longitudinal edges of the absorbent core, and an adjustment edge attached, by means of a second joint, which in turn consists of four joints: two in the upper longitudinal ends and two in the lower longitudinal ends, to the topsheet of the article, such that the second joint is oriented towards the longitudinal edges of the article.

## Description

### Background of the Invention

Disposable absorbent articles have become an essential item in today's day-to-day lives of families. As a result, a multiplicity of technical proposals tending to improve such articles have been developed. However, notwithstanding substantial efforts in connection with research and development, some problems still persist. Among them, it is noteworthy the problem related to lateral leakage, mainly leakage of liquid and semi-liquid materials.

Several proposals have arisen concerning solutions to lateral leakage, among which it is noteworthy the use of anti-leakage lateral cuffs, which can be inner cuffs or outer cuffs. The inner cuffs to stop the flow of exudates which penetrate the absorbent core and have a tendency to exit through the lateral edges thereof, and outer cuffs to stop liquid or semi-liquid exudates that are not rapidly absorbed and remain floating on the topsheet of the diaper. Among the outer cuff systems that have been developed are those of MX 161,292; MX 161,320; and MX 185,778. The cuffs disclosed in these patents are disposed on the topsheet of the diaper, having an edge fixed to the topsheet thereof and an elastic element disposed in the other edge (adjustment edge), to space same from the topsheet. Generally, they are disposed adjacent the lateral edges of the absorbent core, in the crotch zone, over the topsheet of the diaper. In all these systems, the upper and lower longitudinal ends of the cuffs are joined at the adjustment edge to the topsheet of the diaper at the front and back waist parts. This joint is generally oriented towards the center thereof, such that, upon raising the central part of the cuff due to the tension of the elastic member at the adjustment edge, a containment channel is formed for exudates. It is recommended, when putting the diaper on the user, that the cuffs are open so that the cuffs are raised and the containment channel is formed. However, the use of these designs in commercial articles has shown they have a problem, when the diaper is put on without proper care, in that the cuffs collapse and remain over the topsheet, without creating the containment channel. On the other hand, generally the crotch portion of the diaper is the narrowest thereof and is comprised of the crotch portion of the absorbent core and two lateral flaps at each side of the absorbent core, each of the flaps containing the leg elastic member(s). If the diaper includes anti-leakage cuffs, either disposed adjacent the longitudinal edges or the absorbent core or between the leg elastic members, and the cuffs are disposed according to the disclosure of the cited patents, there is a risk that the adjustment edges thereof remain disposed very close to each other, such that they could hurt the genitals of the user, or at least cause discomfort to the user.

Due to the foregoing, the present invention proposes a type of anti-leakage outer cuffs with an open configuration, such that each of the outer cuffs has a fixed edge attached to the topsheet along the entire length of the diaper in an adjacent manner and, preferably, over the longitudinal edges of the absorbent core in the crotch portion and an adjustment edge which is attached to the topsheet at its upper and lower longitudinal ends and at the front and back waist portions of the diaper; this joint is oriented towards the longitudinal edges of the diaper, that is the outer cuffs remain with an open configuration, having a distance between the adjustment edges of the outer cuffs at least approximately equal to the width of the absorbent core in the central portion thereof, such that due to the configuration taken by the diaper when put on the user, the outer cuffs in the crotch portion are disposed parallel to the lateral flaps of the diaper, thus forming an actual wall that stops the passage of liquid, solid or semi-liquid exudates towards the longitudinal edges of the diaper, and there is in addition a greater exudate containment area.

There have been other attempts to try to make anti-leakage cuffs that actually stop the exudates, such as the system disclosed in US 6,183,459, in which the cuffs have a semi-circle shape and are made of an elastic material such that they actually acquire a position perpendicular to the absorbent core when the diaper is fit in the user. The use of cuffs of this type requires costlier materials, as well as a complex manufacturing process.

### Obiects of the Invention

An object of the present invention is to provide a solution to the lateral leakage problem in disposable absorbent articles.

Another object of the invention is to seek a solution of the cited problem which is feasible, inexpensive and easy to apply.

An additional object of the invention consists of providing anti-leakage outer cuffs that are comfortable and do not cause damage or discomfort to the user.

Another object of the invention consists of cuffs which, during use, are disposed in a perpendicular fashion with regard to the absorbent core, forming a retention wall that prevents passage of exudates through same.

Another object is to have the distance between the adjustment edges of the cuffs, when the diaper is fit on the user, to be at least approximately equal to the width of the absorbent core in the crotch portion.

### Brief Description of the Drawings

Figure 1 shows a diaper with all its components, including the cuffs in accordance with the teachings of the present invention.
Figure 2 is a cross-section along line A-A' in Figure 1.
Figure 3 is a perspective view of a diaper, showing the diaper as it would be seen prior to being fit on the user.
Figure 4 illustrates an embodiment of a diaper which uses the same material of the topsheet to form the outer cuffs.
Figure 5 illustrates another embodiment of the outer cuffs of the present invention.

### Detailed Description of the Invention

The term "disposable absorbent article" shall mean in this specification articles that absorb and contain body exudates and which are discarded after use. The present specification will be made based on a disposable diaper for babies, it being understood that the invention can be applied to other types of disposable absorbent articles, such as disposable diapers for incontinent adults, training pants and feminine sanitary napkins.

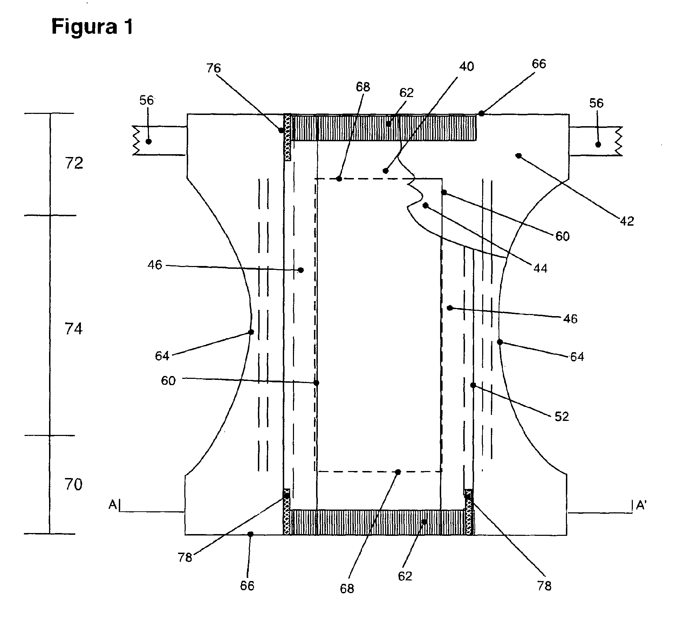

Referring to Figure 1, it shows a disposable diaper formed by a topsheet (40) pervious to liquids, a backsheet (42) impervious to liquids, an absorbent core (44) disposed between both the topsheet and the backsheet, a pair of tape tabs (56) adhered to the topsheet and/or backsheet in the back waist portion of the diaper and which, together with the band(s) (58) which are in the front portion and over the topsheet, form a diaper fastening system, and one or more waist elastic members (62). The absorbent core (44) has to transverse edges (68) and two longitudinal edges (60). In an embodiment of the invention, the topsheet (40) and backsheet (42) are coterminous and extend beyond the margins of the absorbent core (44), thus forming the periphery of the diaper. All the components of the diaper are attached using conventional techniques which are well known by those skilled in the art. As an example, the components can be attached by means of thermal or ultrasonic joints; however, the most common method to attach them involves the use of pressure-sensitive, hot-melt adhesives.

The absorbent article of the present invention has a frontal zone (70), a back zone (72) and a crotch zone (74); the front zone (72) will be disposed during use in the frontal waist and hip portions of the user, the back zone (72) will be disposed in the back waist and hip zones thereof, while the crotch zone (74) is the one that will be disposed during use between the legs of the user. On the other hand, the articles has two longitudinal edges (64) and two transverse edges (66) which border same. Figure 1 shows a rectangular absorbent core (44); however, same could have other shapes, such as a hour-clock shape, an "I" shape, a "T" shape, or any other shape deemed to be convenient.

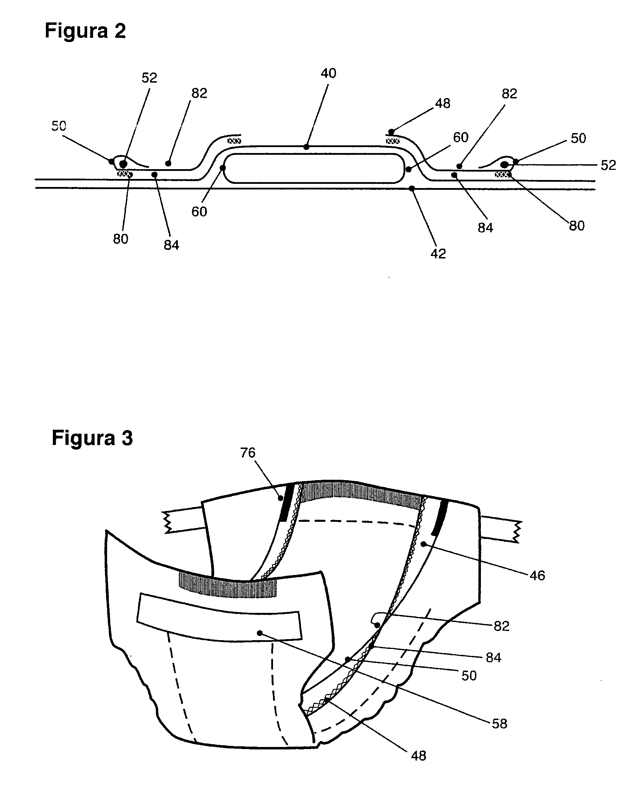

The diaper of the present invention also has two anti-leakage, lateral outer cuffs (46), which are comprised of a hydrophobic material or film which has two faces, an inner face (82) that will be in contact with body exudates and an outer face (84) where the joints or attachments to the topsheet will be disposed, as can be seen in Figure 2, which is a cross-section along lines A-A' in Figure 1; the outer cuffs (46) are disposed over the topsheet adjacent and preferably over the longitudinal edges (60) of the absorbent core. The outer cuffs (46) have a fixed edge (48), attached at the outer face (84) of the film, at the topsheet (40), and an adjustment edge (50) which during use raises and fits to the body of the user; in the adjustment edge (50) an elastic element (52) is disposed with a certain elongation, the function of which is to raise the adjustment edge (50) of each outer cuff, at least in the crotch zone (74) such that when the diaper adopts its usual shape, as disposed in the user (Figure 3), the outer cuffs (46) raise and are disposed in a perpendicular fashion with regard to absorbent core (44) and to topsheet (40), at least in the crotch zone (74) of the diaper, forming a wall that will prevent passage of liquid, semi-liquid or solid exudates towards the longitudinal edges (64) of the diaper. Fixed edge (48) of each of the outer cuffs will be attached to the topsheet of the diaper, by means of a first joint, adjacent and preferably over the longitudinal edges (60) of the absorbent core in the crotch zone thereof, using any system known in the art, such as hot-melt adhesive, ultrasound, etc. The adjustment edge (50) of the outer cuff is attached, by means of a second joint (80), at the outer face (84) of the film, to the topsheet (40) only in the upper and lower portions thereof, in the back (72) and frontal (70) portions of the diaper. This second joint is oriented towards the longitudinal edges (64) of the diaper, that is, the cuffs are open, leaving free the central portion of the core, such that there is no risk the elastic member (52) located at the adjustment edges hurt or cause discomfort to the genitals of the user, since said elastic members (52) will conform to the groin of the user in the crotch portion or zone.

On the other hand, the second joint (80) of the cuffs is formed by four joints, two joints (78) in the frontal zone (70) of the article and two joints (76) in the back zone (72) thereof. Each of these joints has a maximum length such that the distance which remains free between the second joint (78) in the frontal portion and the second joint (76) in the back portion of the article is at least equal to approximately one third of the total length of the article, thus permitting the outer cuffs to be free and adapt to the body of the user in the crotch zone thereof.

The distance between the elastic members of the adjustment edges of said outer cuffs, when the diaper takes a configuration suitable for use, such as that shown in Figure 3, is at least approximately equal to the width of the absorbent core in the crotch zone.

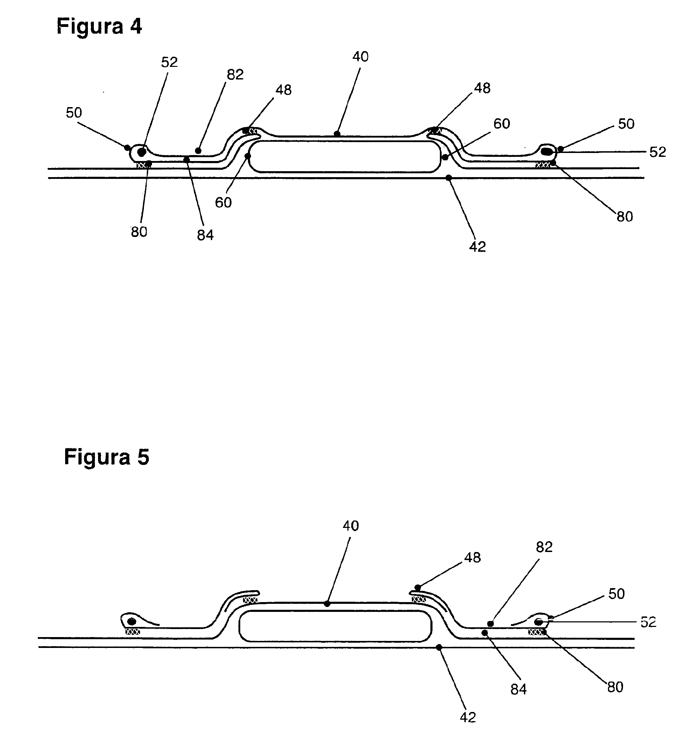

The outer cuffs (46) of the present invention are made of a material different from that of topsheet (40) of the diaper; preferably, they are breathable and hydrophobic; however, in an embodiment of the invention, they can be made of the same material as the topsheet, such as shown in Figure 4. In this case, the first joint (48) joins two portions of the topsheet to form the cuff, such as can be seen in Figure 4, and the second joint attaches the fixed edge to the same topsheet at the front and back portions of the article, in a joint oriented towards the longitudinal edges thereof.

The height of outer cuffs (46) can vary as a function of the particular product; as an example, they are higher in an adult diaper than in a baby diaper. However, their height is limited by the longitudinal edges (64) of the diaper in the crotch portion (74), since the outer cuffs will have a maximum height equal to the distance between the longitudinal edges of the absorbent core in the crotch portion and the longitudinal edges of the diaper in the crotch portion plus about 15 mm.

Figure 5 shows another embodiment of the outer cuffs in accordance with the present invention, wherein outer cuffs (46) are attached by means of their fixed edge (48) to the topsheet (40) of the diaper in a manner adjacent the longitudinal edges (60) of the absorbent core in the inner face (82) of the film which forms same and oriented towards the longitudinal edges (64) of the diaper, attaching the adjustment edge (50) of each of the cuffs in the outer face (84) of the film forming same, to the topsheet, in the upper (76) and lower (78) portions thereof, in the back (72) and frontal (70) zones of the diaper.

While the invention has been described as a function of a currently preferred embodiment, it is apparent that changes and variations thereof can be made, all such changes and variations falling with the scope and spirt of the invention, as defined in the appended claims.

## Claims

1. A disposable absorbent article, comprising a pervious topsheet, an impervious backsheet, an absorbent core disposed between the topsheet and the backsheet, the absorbent core having two longitudinal edges and two transverse edges, elastic waist bands and a fastening system, the article having a front zone, a back zone and a crotch zone, as well as two longitudinal edges and two transverse edges; **characterized in that** it has two anti-leakage outer cuffs disposed over the topsheet and formed by means of a film having an outer face and an inner face, the outer cuffs having a fixed edge attached, by means of a first joint, in the outer face of the film forming same, along the entire length of the diaper adjacent and preferably over the longitudinal edges of the absorbent core in the crotch zone of the article, and an adjustment edge attached, by means of a second joint which in turn consists of four joints: two joints in the front zone of the article and two joints in the back zone thereof, to the topsheet of the article, such that the second joint is oriented towards the longitudinal edges thereof.

2. A disposable absorbent article, comprising a pervious topsheet, an impervious backsheet, an absorbent core disposed between the topsheet and the backsheet, the absorbent core having two longitudinal edges and two transverse edges, elastic waist bands and a fastening system, the article having a front zone, a back zone and a crotch zone, as well as two longitudinal edges and two transverse edges; **characterized in that** it has two anti-leakage outer cuffs disposed over the topsheet and formed by means of a film having an outer face and an inner face, the outer cuffs having a fixed edge attached, by means of a first joint, in the inner face of the film forming same, along the entire length of the diaper adjacent and preferably over the longitudinal edges of the absorbent core in the crotch zone of the article, and an adjustment edge attached, by means of a second joint which in turn consists of four joints: two joints in the front zone of the article and two joints in the back zone thereof, to the topsheet of the article, such that the second joint is oriented towards the longitudinal edges thereof.

3. A disposable absorbent article, as recited in any of claims 1 and 2, **characterized in that** the distance which remains free between the second front joint and the second back joint of each of the outer cuffs has a minimum length equal to about one third of the entire length of the article.

4. A disposable absorbent article, as recited in any of claims 1 and 2, **characterized in that** the maximum height of the outer cuffs is approximately equal to the distance between the longitudinal edges of the absorbent core and the longitudinal edges of the article in the crotch zone.

5. A disposable absorbent article, as recited in any of claims 1 and 2, **characterized in that** the maximum height of the outer cuffs is approximately equal to the distance between the longitudinal edges of the absorbent core and the longitudinal edges of the article in the crotch zone plus about 15 mm.

6. A disposable absorbent article, as recited in any of the preceding claims, **characterized in that** the distance between the adjustment edges of the outer cuffs when the articles takes a configuration convenient for use is at least approximately equal to the width of the absorbent core in the crotch zone.

7. A disposable absorbent article, as recited in any of the preceding claims, **characterized in that** the film that forms the anti-leakage outer cuffs is of a material different from the material of the topsheet of the article.

8. A disposable absorbent article, as recited in any of claims 1 and 6, **characterized in that** the film forming the anti-leakage outer cuffs is of the same material as the topsheet of the article.

9. A disposable absorbent article, comprising a pervious topsheet, an impervious backsheet, an absorbent core disposed between the topsheet and the backsheet, the absorbent core having two longitudinal edges and two transverse edges, elastic waist bands and a fastening system, the article having a front zone, a back zone and a crotch zone, as well as two longitudinal edges and two transverse edges; **characterized in that** it has two anti-leakage outer cuffs formed from the pervious topsheet of the article, the outer cuffs having a fixed edge attached along the entire length of the diaper adjacent and preferably over the longitudinal edges of the absorbent core in the crotch zone of the article which join, by means of a first joint, two portions of the topsheet to form the cuffs, and an adjustment edge attached, by means of a second joint which in turn consists of four joints: two joints in the front zone of the article and two joints in the back zone thereof, to the topsheet of the article, such that the second joint is oriented towards the longitudinal edges thereof.

10. A disposable absorbent article, as recited in claim 9, **characterized in that** the distance that remains free between the second front joint and the second back joint in each of the cuffs has a minimum length equal to approximately one third of the entire length of the article.

11. A disposable absorbent article, as recited in claim 9, **characterized in that** the maximum height of the cuffs is approximately equal to the distance between the longitudinal edges of the absorbent core and the longitudinal edges of the article in the crotch zone.

12. A disposable absorbent article, as recited in claim 9, **characterized in that** the maximum height of the cuffs is approximately equal to the distance between the longitudinal edges of the absorbent core and the longitudinal edges of the article in the crotch zone plus about 15 mm.

13. A disposable absorbent article, as recited in claim 9, **characterized in that** the distance between the adjustment edges of the cuffs, when the diaper takes a configuration convenient for use, is at least approximately equal to the width of the absorbent core in the crotch zone thereof.
